# EUROPEAN PATENT APPLICATION

(11) **EP 3 929 586 A1**
(43) Date of publication of application: **29.12.2021**
(21) Application number: 19916107.6
(22) Date of filing: 13.11.2019
(51) Int. Cl.: G01N 33/574, G01N 33/564, G01N 33/58, G01N 33/68, C07K 16/30

(54) **IMMUNOLOGICAL COMPOSITION FOR DIAGNOSIS OF LUNG CANCER BY USING AUTOANTIBODY-ANTIGEN COMPLEX, DIAGNOSIS METHOD FOR LUNG CANCER BY USING SAME, AND LUNG CANCER DIAGNOSIS KIT COMPRISING SAME**

(30) Priority: 22.02.2019 KR 20190021469
(71) Applicant: Biometrix Technology Inc., Gangwon-do 24232 (KR)
(72) Inventor: KIM, Taisun, Chuncheon-si Gangwon-do 24375 (KR); SONG, Keumsoo, Chuncheon-si Gangwon-do 24419 (KR); KIM, Junghun, Seoul 03081 (KR)
(74) Representative: Gulde & Partner
(86) International application number: PCT/KR2019/015477
(87) International publication number: WO 2020/171346

(57) **Abstract**

The present disclosure provides an immunological composition for diagnosing lung cancer comprising: an antibody composition A containing an anti-CYFRA21-1 primary antibody-gene and an anti-CYFRA21-1 secondary antibody-detection marker; and an antibody composition B containing an anti-CYFRA 21-1 primary antibody-gene and an anti-human IgG antibody-detection marker; a method for diagnosing lung cancer in a human biological specimen using the same, and a diagnostic kit for lung cancer using the same.

The composition for diagnosing lung cancer and the diagnostic method for lung cancer according to the present disclosure can diagnose the onset of lung cancer stages 0 to IV in a non-invasive biological specimen with a specificity of 90% or more and a sensitivity of 75% or more. In particular, since an autoantibody whose concentration increases rapidly in the early stage of lung cancer is used, it is possible to diagnose the onset of lung cancer stages 0 to 1 with a sensitivity of 76% or more.

## Description

### [TECHNICAL FIELD]

The present disclosure relates to an immunological composition for diagnosing lung cancer using an autoantibody-antigen conjugate, a method for diagnosing lung cancer using the same, and a diagnostic kit for lung cancer comprising the same

### [BACKGROUND ART]

It is internationally known that almost one million people die from lung cancer annually. Diagnosis and treatment techniques have been developed over the last few decades, but there are no special subjective symptoms, most of which are discovered in the stage of progressive cancers or in the stage where it has metastasized to other sites. Thus, the rate of complete recovery of lung cancer patients is less than 30% and the prognosis is very poor.

If even these lung cancers can be diagnosed at early stage, the survival rate can be dramatically increased by 80% or more. However, currently, it is highly dependent on imaging methods (X-ray, CT, MRI, etc.) for diagnosis of lung cancer, and the cases in which materials capable of being used as biochemical markers were found is rare.

At present, the materials proposed under the name of a diagnostic biomarker for lung cancer exist, but they do not yet exhibit sufficient specificity and sensitivity.

Therefore, in relation to the prevalence of lung cancer, which is rapidly increasing at home and abroad, it is essential to secure a source technology for the development of lung cancer biomarkers and diagnostic kits mainly in advanced countries, and there is an urgent need to develop a new diagnostic method for lung cancer that can detect lung cancer at early stage (stages 0 to I).

The method of diagnosing lung cancer includes the following three:
1) Imaging method (X-ray, CT, MRI): Currently, it is highly dependent on the imaging method in order to diagnose lung cancer, but sensitivity and specificity are not good for early diagnosis of lung cancer.
2) Biopsy: Biopsy includes sputum cell assay, bronchoscopy, fine-needle aspiration biopsy, mediastinoscopy and the like. As the pathological and cytological detection method requiring biopsy tissue, it is invasive and the repetitive measurement is difficult, which is thus disadvantageous.
3) Tumor marker test: A non-invasive diagnostic method for diagnosing malignant tumor, which is widely used as a favorable factor for predicting the prognosis and determining therapeutic effect. Among them, CYFRA21-1 is a fragment of cytokeratin 19, and since its presentation as a lung cancer marker in 1993, studies on its utility have been ongoing. In many studies, it is recognized as a marker for non-small cell lung cancer. However, since most of tumor markers use a cutoff value that judges as cancer when the concentration exceeds a certain level, and judges as normal if it is not, they have limitations in the accuracy of distinguishing between normal human and cancer patients.

Even if it exceeds the normal cut off value, it cannot be said that it is a cancer, and even if it is below the normal cut off value, it cannot be said that it is not a cancer. Therefore, in order to clearly diagnose cancer, precise examination such as biopsy is necessary.

Tumor marker tests are based on antigen detection methods, and studies on tumor markers have been continuously conducted, but using only the tumor marker tests, quantitative changes of tumor markers in blood appear to be very small from stage 0 to stage III cancer, and a quantitative difference appears in approximately stage IV. Therefore, a test using only an antigen, which is a tumor marker with a slight quantitative change, shows a low specificity of 89% and a sensitivity of 43%, which makes it difficult to distinguish between normal and cancer. In particular, it is very difficult to distinguish between normal and cancer at cancer stages 0 to I.

Therefore, in order to distinguish between normal and cancer, there is a need for a new tumor marker which shows a very large quantitative change in blood between normal and cancer, and a new detection and diagnostic method using the same.

### [DETAILED DESCRIPTION OF THE INVENTION]

### [Technical Problem]

Autoantibody that reacts specifically with an antigen appears a quantitative increase when cancer occurs. Because the immune system is active in the early stage of cancer (stage 0 to I), the quantitative increase of the autoantibody appears the largest, and shows the width of increase of 5 to 10 times more compared to normal.

The present disclosure is intended to develop a method in which in the case of cancer, autoantibodies showing a very large quantitative increase in blood compared to normal were measured to distinguish between normal and cancer.

### [Technical Solution]

In order to achieve the above objects, one aspect of the present disclosure provides an immunological composition for diagnosing lung cancer comprising: an antibody composition A containing an anti-CYFRA21-1 primary antibody-gene and an anti-CYFRA21-1 secondary antibody-detection marker; and an antibody composition B containing an anti-CYFRA 21-1 primary antibody-gene and an anti-human IgG antibody-detection marker.

Another aspect of the present disclosure provides a method for diagnosing lung cancer in a human biological specimen using the above-mentioned immunological composition for diagnosing lung cancer, the method comprising the steps of:
(A) measuring a concentration of CYFRA21-1 antigen using the antibody composition A;
(B) measuring a concentration of anti-CYFRA21-1 autoantibody-antigen conjugate using the antibody composition B; and
(C) determining normal and lung cancer in the biological specimen from the ratio of the concentration obtained in the above (a) and the concentration obtained in the above (b).

### [ADVANTAGEOUS EFFECTS]

The immunological composition for diagnosing lung cancer and the diagnostic method for lung cancer according to the present disclosure can diagnose the onset of lung cancer stages 0 to IV in a non-invasive biological specimen with a specificity of 90% or more and a sensitivity of 75% or more. In particular, since an autoantibody whose concentration increases rapidly in the early stage of lung cancer is used, it is possible to diagnose the onset of lung cancer stages 0 to 1 with a sensitivity of 76% or more.

### [BRIEF DESCRIPTION OF THE DRAWINGS]

FIG. 1 is a graph showing the ratio of <anti-CYFRA21-1 autoantibody-antigen conjugate> and <CYFRA21-1 antigen> obtained using the autoantibody-antigen conjugate and antigen detection methods of the present disclosure in normal human and lung cancer patients.
FIG. 2 is a conceptual diagram showing that <anti-CYFRA21-1 primary antibody-gene> and <anti-CYFRA21-1 secondary antibody-detection marker> bind to <CYFRA21-1 antigen> in a biological specimen to form a complex of <anti-CYFRA21-1 primary antibody-gene>: <CYFRA21-1 antigen>: <anti-CYFRA21-1 secondary antibody-Cy5>.
FIG. 3 is a conceptual diagram showing that <anti-CYFRA21-1 primary antibody-gene> and <anti-human IgG antibody-Cy5> bind to <anti-CYFRA21-1 autoantibody-antigen> in a biological specimen to form a complex of <anti-CYFRA21-1 primary antibody-gene>:<anti-CYFRA21-1 autoantibody-antigen conjugate>:<anti-human IgG antibody-Cy5>.
FIG. 4 illustrates a mode for detecting CYFRA21-1 antigen on a 9G DNA membrane.
FIG. 5 illustrates a mode for detecting anti-CYFRA21-1 autoantibody-antigen conjugate on a 9G DNA membrane.
FIG. 6 is a graph showing quantitative changes in blood of cancer antigen (Ag) and autoantibody (AAb) according to normal and lung cancer stages (stages 0 to III).
FIG. 7 is a graph for the concentration of antigen in an antigen test (ELISA method), which shows difficulty in distinguishing between normal and cancer patients.

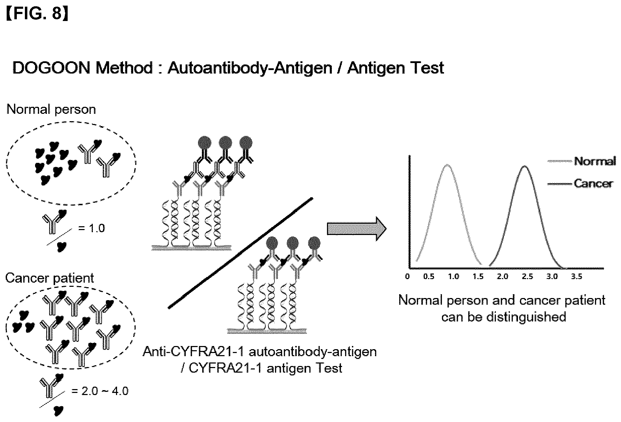

FIG. 8 is a graph for the concentration of <autoantibody-antigen conjugate> and <antigen> according to the present disclosure, which shows that normal and cancer patients can be easily distinguished since <anti-CYFRA21-1 autoantibody-antigen conjugate> and <CYFRA21-1 antigen> appear in different regions.
FIG. 9 shows a schematic diagram of an antibody composition A containing <anti-CYFRA21-1 primary antibody-gene> and <anti-CYFRA21-1 secondary antibody-Cy5>; and a schematic diagram of an antibody composition B containing <anti-CYFRA21-1 primary antibody-gene> and <anti-human IgG antibody-Cy5>, respectively.
FIG.10 is a conceptual diagram showing a process in which <CYFRA21-1 antigen> is detected when the diagnostic method for lung cancer according to the present disclosure has been performed on a 9G DNA membrane.
FIG.11 is a conceptual diagram showing a process in which <anti-CYFRA21-1 autoantibody-antigen conjugate> is detected when the diagnostic method for lung cancer according to the present disclosure has been performed on a 9G DNA membrane.
FIG. 12 shows a mode for applying the diagnostic kit for lung cancer according to the present disclosure to a 9G DNA membrane.
FIG. 13 is a photograph showing a process of detecting and reading a diagnostic kit for lung cancer applied to a 9G DNA membrane.
FIG. 14 is a graph showing hospital clinical results obtained by measuring lung cancer sensitivity by applying the diagnostic composition for lung cancer according to the present disclosure to 200 normal specimens and 50 lung cancer (stages I to IV) specimens.

### [DETAILED DESCRIPTION OF THE EMBODIMENTS]

The present disclosure provides an immunological composition for diagnosing lung cancer comprising: an antibody composition A containing an anti-CYFRA21-1 primary antibody-gene and an anti-CYFRA21-1 secondary antibody-detection marker; and an antibody composition B containing anti-CYFRA 21-1 primary antibody-gene and anti-human IgG antibody-detection marker.

According to one embodiment of the present disclosure, the detection marker may be any one selected from the group consisting of a chromogenic enzyme, a fluorescent material, a fluorescent bead, a radioactive isotope, and a colloid, and preferably, it may be selected from a fluorescent material.

The present disclosure provides a method for diagnosing lung cancer in a human biological specimen using the above-mentioned immunological composition for diagnosing lung cancer, the method comprising the steps of:
(A) measuring a presence or absence or a concentration of <CYFRA 21-1 antigen> in the biological specimen by using an antibody composition A containing <anti-CYFRA21-1 primary antibody-gene> and <anti-CYFRA21-1 secondary antibody-detection marker>,
(B) measuring a presence or absence or a concentration of <anti-CYFRA 21-1 autoantibody-antigen conjugate> in the biological specimen by using an antibody composition B containing <anti-CYFRA 21-1 primary antibody-gene> and <anti-human IgG antibody-detection marker>; and
(C) determining normal and lung cancer in the biological specimen from the ratio of the concentration obtained in the above (a) and the concentration obtained in the above (b).

According to one embodiment of the present disclosure, it is possible to determine whether the biological specimen is normal status or lung cancer status in by using the ratio of a complex of <anti-CYFRA21-1 primary antibody-gene>: <CYFRA 21-1 antigen>:<anti-CYFRA21-1 secondary antibody-detection marker> formed in the above (a), and a complex of <anti-CYFRA21-1 primary antibody-gene>:<anti-CYFRA21-1 autoantibody-antigen>:<anti-human IgG antibody-detection marker> formed in the above (b).

The present disclosure provides a diagnostic kit for lung cancer comprising the above-mentioned immunological composition for diagnosing lung cancer and a biochip.

The present disclosure relates to a method for detecting an anti-CYFRA21-1 autoantibody-antigen conjugate and CYFRA21-1 antigen in a biological specimen, and also to a diagnostic kit and a diagnostic method for diagnosing lung cancer including stages 0 to I by using the ratio of anti-CYFRA21-1 autoantibody-antigen conjugate and CYFRA21-1 antigen.

An object of the present disclosure is to provide a method for detecting a CYFRA21-1 antigen in a biological specimen and an anti-CYFRA 21-1 autoantibody-antigen conjugate in a form bound thereto, and using the same.

Therefore, a first object of the present disclosure is to provide an immunological composition for diagnosing lung cancer comprising: an antibody composition A containing an anti-CYFRA21-1 primary antibody-gene and an anti-CYFRA21-1 secondary antibody-detection marker; and an antibody composition B containing an anti-CYFRA 21-1 primary antibody-gene and an anti-human IgG antibody-detection marker.

In some embodiments, the detection marker can be detected by a method such as color development, light emission, or fluorescence.

According to some embodiments of the present disclosure, the detection marker may be any one selected from the group consisting of a fluorescent material, a fluorescent bead, a radioactive isotope, and a colloid. The above-mentioned colloid is generally a marker for visual detection, and for example, a nano-metal colloid such as a nano gold colloid may be used.

According to some embodiments of the present disclosure, the detection marker can be selected from fluorescent materials. For example, cyanine Cy3 (570 nm emission), Cy5 (670 nm emission), FAM, VIC, TET, JOE, HEX, ROX, RED610, TEXAS RED, RED670, TYE563, and NED can be used.

A second object of the present disclosure is to provide a method for diagnosing lung cancer in a human biological specimen using the above-mentioned immunological composition for diagnosing lung cancer, the method comprising the following steps:
(A) measuring a concentration of <CYFRA21-1 antigen> in the biological specimen using an antibody composition A containing <anti-CYFRA21-1 primary antibody-gene> and <anti-CYFRA21-1 secondary antibody-detection marker>;
(B) measuring a concentration of <anti-CYFRA21-1 autoantibody-antigen conjugate> in the biological specimen using an antibody composition B containing <anti-CYFRA21-1 primary antibody-gene> and <anti-human IgG antibody-detection marker> ; and
(C) determining normal and lung cancer in the biological specimen from the ratio of the concentration obtained in the above (a) and the concentration obtained in the above (b).

According to an embodiment of the present disclosure, in the above (a), <anti-CYFRA21-1 primary antibody-gene> and <anti-CYFRA21-1 secondary antibody-detection marker> contained in the antibody composition A can be bound to <CYFRA21-1 antigen> in a biological specimen to form a complex of <anti-CYFRA21-1 primary antibody-gene>:<CYFRA21-1 antigen>:<anti-CYFRA21-1 secondary antibody-detection marker>, thereby measuring the presence or absence or the concentration of <CYFRA21-1 antigen>.

According to one embodiment of the present disclosure, in the above (b), <anti-CYFRA21-1 primary antibody-gene> and <anti-human IgG antibody-detection marker> contained in the antibody composition B are bound to <anti-CYFRA21-1 autoantibody-antigen conjugate> in the biological specimen to form a complex of <anti-CYFRA21-1 primary antibody-gene>:<anti-CYFRA21-1 autoantibody-antigen>:<anti-human IgG antibody-detection marker>, thereby measuring the presence or absence or the concentration of <anti-CYFRA21-1 autoantibody-antigen conjugate>.

According to one embodiment of the present disclosure, in the above (c), it is possible to determine whether the biological specimen is in a normal state or in a lung cancer onset state, by using the ratio of a complex of <anti-CYFRA21-1 primary antibody-gene>:<CYFRA21-1 antigen>:<anti-CYFRA21-1 secondary antibody-detection marker> and a complex of <anti-CYFRA21-1 primary antibody-gene>:<anti-CYFRA21-1 autoantibody-antigen>:<anti-human IgG antibody-detection marker>, and in some cases, it is possible to determine the progression of lung cancer

According to one embodiment of the present disclosure, in the above (c), when the ratio of <anti-CYFRA21-1 autoantibody-antigen conjugate>/< CYFRA21-1 antigen> is higher than a value that can be selected from a certain cutoff value, for example, 1 or more, preferably 1.5 or more, more preferably 2 or more, specifically 1 to 4, and more specifically 1.5 to 3, it can be selected as lung cancer, or can be determined to be in the onset or progression state of lung cancer

According to an embodiment of the present disclosure, the biological specimen may be any one selected from the group consisting of whole blood, serum, plasma, saliva, urine, sputum, lymph fluid, cerebrospinal fluid, and cell fluid.

According to an embodiment of the present disclosure, the antigen-antibody complexes formed in the above (a) and (b) may be immobilized on a solid support.

According to an embodiment of the present disclosure, the solid support may include a 9G membrane or a 9G DNA membrane.

According to an embodiment of the present disclosure, the antigen specific for the anti-CYFRA21-1 autoantibody may be a CYFRA21-1 protein.

In addition, the immune complex may be further bound to the <anti-CFYRA21-1 autoantibody-antigen> conjugate. The <anti-CFYRA21-1 autoantibody-antigen> conjugate is a human autoantibody-antigen conjugate, and the molecule that binds to the anti-CFYRA21-1 autoantibody-antigen conjugate is an anti-human IgG antibody.

The present disclosure is intended to discover lung cancer at stages 0 to I through blood measurement, which is a non-invasive method, by using a platform to which DAGON technology (Chemical Communications 2011, 47, 7616) based on 9G DNA technology (Chemical Communications 2011, 47, 7104) is applied, thereby increasing the survival rate and improving the quality of life.

Therefore, a third object of the present disclosure is to provide a diagnostic kit for lung cancer comprising the above-mentioned immunological composition for diagnosing lung cancer and a biochip.

According to an embodiment of the present disclosure, the biochip may include a 9G membrane or a 9G DNA membrane.

As used herein, the term "9G membrane" means a glass fiber membrane in which a surface on which an oligo gene having continuous 9 guanines is immobilized is modified with a calixarene derivative, and the term "9G DNA membrane" means a glass fiber membrane in which an oligo gene having 9 consecutive guanines is immobilized. The "9G membrane" and "9G DNA membrane" are based on the above-mentioned 9G DNA technology (Chemical Communications 2011, 47, 7104).

Hereinafter, the present disclosure will be described in more detail with reference to examples and drawings, but the present disclosure is not limited thereto.

The present disclosure provides a method for distinguishing between normal and cancer using a quantitative ratio with an antigen by measuring an autoantibody which shows that in the case of cancer, the quantitative increase in blood is very high compared to normal.

When lung cancer occurs, the CYFRA21-1 antigen, which is a cancer-related protein showing a quantitative increase in blood, and the anti-CYFRA21-1 autoantibody, which react specifically with this CYFRA21-1 antigen exhibit an increase in quantity, wherein the anti-CYFRA21-1 autoantibody exists as an anti-CYFRA21-1 autoantibody-antigen conjugate which is a form bound to an antigen.

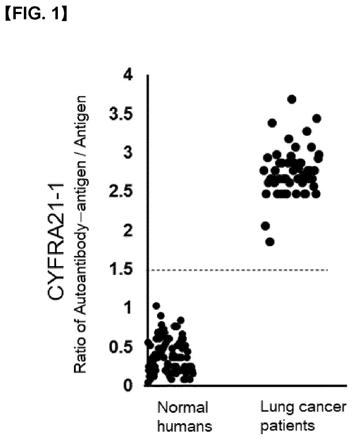

The present disclosure provides a method in which the amount of CYFRA21-1 antigen with little quantitative increase in cancer stages 0 to I and the amount of the antigen-conjugate bound to the anti-CYFRA21-1 autoantibody with a very large quantitative increase can be accurately detected, thereby detecting normal or cancer with an early stage by utilizing these quantitative ratio (see FIG. 1).

In order to distinguish between normal and cancer at cancer stages 0 to I, quantitative changes in antigens and autoantibodies in a biological specimen must be accurately measured, and there should be no interference phenomena and non-specific reactions in the specimen.

The present disclosure prepares a diagnostic kit for lung cancer having no interference and non-specific reaction using a glass fiber immobilized with a gene prepared by the method already known in the art (Korean Patent Nos. 10-0883763 and 10-1682347) , and provide a method of using the same.

An object of the present disclosure is to detect a CYFRA21-1 antigen in a biological specimen and an anti-CYFRA21-1 autoantibody-antigen conjugate bound thereto, and provide a method using the same.

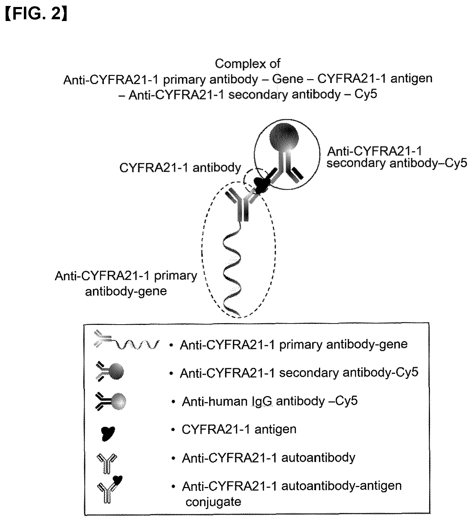

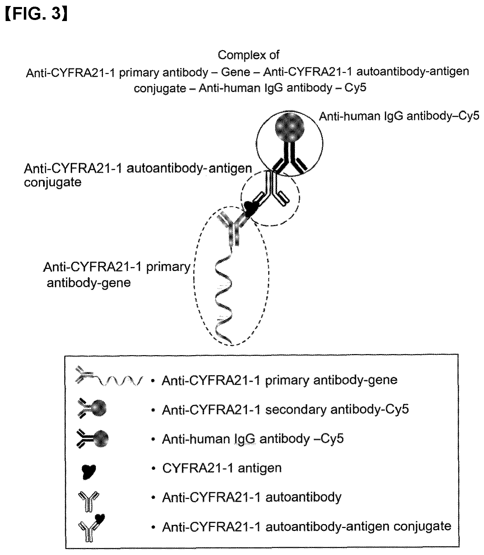

The present disclosure relates to a fluorescence immunoassay method for detecting an anti-CYFRA21-1 autoantibody-antigen conjugate and a CYFRA21-1 antigen. Specifically, the present disclosure provides an immunoassay method for assaying the presence or absence and ratio of anti-CYFRA21-1 autoantibody-antigen conjugate and CFYRA21-1 antigen in human biological specimens, the method comprising the steps of:
(A) assaying the presence or absence or the concentration of CYFRA 21-1 antigen using the antibody composition A: in this step, (anti-CYFRA21-1 primary antibody-gene) and (anti-CYFRA21-1 secondary antibody-Cv5) bind to (CYFRA21-1 antigen) in a biological specimen to form a complex of (anti-CYFRA21-1 primary antibody-gene):(CYFRA21-1 antigen):(anti-CYFRA21-1 secondary antibody-Cv5) (FIG. 2).
(B) assaying the presence or absence or the concentration of the anti-CYFRA21-1 autoantibody-antigen conjugate using the antibody composition B: in this step, (anti-CYFRA21-1 primary antibody-gene) and (anti-human IgG antibody-Cv5) bind to (anti-CYFRA21-1 autoantibody-antigen conjugate) in the biological specimen to form a complex of (anti-CYFRA21-1 primary antibody-gene):(anti-CYFRA21-1 autoantibody-antigen):(anti-human IgG antibody-Cy5) (FIG. 3).
(C) determining normal and lung cancer in the biological specimen using the ratio of a complex of (anti-CYFRA21-1 primary antibody-gene):(anti-CYFRA21-1 autoantibody-antigen):(anti-human IgG antibody-Cv5) and a complex of (anti-CYFRA21-1 primary antibody-gene):(CYFRA21-1 antigen):(anti-CYFRA21-1 secondary antibody-Cv5) formed above.

The present disclosure provides a diagnostic kit that can diagnose whether non-invasive biological specimens such as blood and plasma from normal and lung cancer patients have progressed into lung cancer stages I to IV with a specificity of 91.7% and a sensitivity of stage I 76%, stage II 80%, stage III 77% and stage IV 50%, by using the ratio of anti-CYFRA21-1 autoantibody-antigen conjugate and CYFRA21-1 antigen, and a method of using the same.

In particular, since it exhibits high diagnostic effect of lung cancer at the level of 76% sensitivity at cancer stages 0 to I, which are the period when autoantibodies rapidly increase, it is very effective in lung cancer diagnosis in stages 0 to I which is the initial stage of lung cancer.

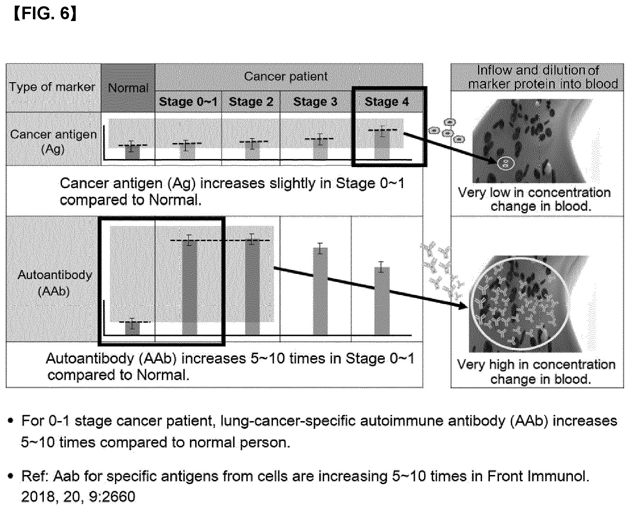

FIG. 6 is a graph showing quantitative changes in blood of cancer antigens (Ag) and autoantibodies (AAb), which are lung cancer tumor markers, according to normal and lung cancer stages (by stage 0∼III).

Referring to FIG. 6, since the cancer antigen (Ag) does not have a large amount of change from a normal state to lung cancer stages 0 to III, and shows a high amount of change only in lung cancer stages III to IV in which lung cancer has progressed considerably, it can be seen that at least at the early stage of lung cancer stages 0 to I, it is not easy to distinguish and diagnose between a normal condition and the onset condition of lung cancer only by an antigen test. On the other hand, since the autoantibody (AAb) increases by 5 to 10 times or more compared to the normal condition in stages 0 to I which is the initial stage of lung cancer, thus showing a clear change of amount, it can be seen that lung cancer can be diagnosed can be diagnosed in the early stage (stages 0 to I).

According to the method of the present disclosure, "anti-CYFRA 21-1 primary antibody-gene" in a state where a gene is bound to an antibody is used so as to accurately detect sensitization of anti-CYFRA21-1 autoantibody-antigen conjugate and CYFRA21-1 antigen marker in a biological specimen, and a complex of antigen-antibody-gene is formed in a solution, and then a solid substrate on which the gene is immobilized and the complex of antigen-antibody-gene are immobilized, and thus, can be immobilized without any interference phenomena or non-specific reactions in biological specimens, so that quantitative changes in markers in blood can be accurately measured.

The following examples are provided to specifically explain the present disclosure, but are not intended to limit the present disclosure.

### <Example 1> Preparation and purification of CYFRA 21-1 primary antibody-gene conjugate

The materials used for the CYFRA21-1 primary antibody-gene conjugate are shown in Table 1 below, and the base sequence of the target gene (TD) used is shown in Table 2 below.

**[Table 1]**

| **Materials used in Example 1** | **Molecular weight** | **Concentration** |
|---|---|---|
| CYFRA21-1 primary antibody | 160,000 g/mol | 7.1 mg/ml, 35 *µℓ* |
| 2-iminothiolane | 137 g/mol | 4 mg/ml |
| sulfo-SMCC | 436 g/mol | 80 mg/ml |
| TD02 | 9000 g/mol | 100 pmol/ml |
| 10×PBS | - | - |
| 500mM EDTA | - | - |

**[Table 2]**

| Target gene | Base sequence |
|---|---|
| TD02 | 5'-NH₂-TTTTTTTTTCCTCCCCAAGTCGTAGG |

The CYFRA21-1 primary antibody-gene conjugate was prepared and purified according to a known method using the materials shown in Table 1, and thereby, 80 *µ*ℓ of CYFRA21-1 primary antibody-gene conjugate was obtained at a concentration of 2 mg/ml. (see Korean Patent Registration No. 10-1682347, Example 1 and FIG. 1)

### <Example 2> Preparation and purification of antibody-detection marker conjugate to which fluorescent labeling material was adhered

A <antibody-detection marker> conjugate was prepared using a fluorescent material as a detection marker, and the materials used for the preparation thereof are shown in Table 3 below.

**[Table 3]**

| **Materials used in Example 2** | **Molecular weight** | **Concentration** |
|---|---|---|
| CYFRA21-1 secondary antibody | 160,000 g/mol | 7.1 mg/ml, 35 *µ*ℓ |
| Anti-human IgG antibody | 160,000 g/mol | 7.6 mg/ml, 33 *µ*ℓ |
| Cy5-bis NHS ester(labeling material) | 436 g/mol | 1 pack in DMSO 20 *µℓ* |
| 10×PBS | - | - |
| 500mM EDTA | - | - |

**[Table 4]**

| | | |
|---|---|---|
| **Materials prepared in Examples 1 and 2** | **Concentration** | **Volume** |
| CYFRA21-1 primary antibody-gene | 2 mg/ml | 80 *µ*ℓ |
| CYFRA21-1 secondary antibody-Cy5 | 2.5 mg/ml | 70 *µ*ℓ |
| Anti-human IgG antibody-Cy5 | 3 mg/ml | 60 *µ*ℓ |

In Table 4, the concentration and volume of the products obtained in Examples 1 and 2 are listed, respectively.

### <Example 3> Preparation of Antibody Compositions A and B for Diagnosing Lung Cancer

Antibody compositions A and B were prepared using the materials listed in Table 5. All antibody concentrations were diluted to 300 µl/ml and used.

**[Table 5]**

| **Antibody composition** | **Material used** | **Concentration** | **Amount used** |
|---|---|---|---|
| **A** | Anti-CYFRA21-1 primary antibody-gene | 300 µg/ml | 4 *µ*ℓ |
| | Anti-CYFRA21-1 secondary antibody-Cy5 | 300 µg/ml | 4 *µ*ℓ |
| | 1XPBS | - | 3.992 ml |
| **B** | Anti-CYFRA21-1 primary antibody-gene | 300 µg/ml | 4 *µ*ℓ |
| | Anti-human IgG antibody-Cy5 | 300 µg/ml | 4 *µ*ℓ |
| | 1XPBS | - | 3.992 ml |

Each constituent material was added in a 5ml tube according to the amount used, and mixed.

### <Example 4> Immobilization of oligo gene for diagnosing lung cancer and preparation of 9G DNA membrane

Table 6 below lists the base sequence of the probe gene.

**[Table 6]**

| Probe name | Base sequence | Concentration |
|---|---|---|
| HC probe | GGGGGGGGG TTT ATA TTT CGGGCAG GCCATAGCGA | 100 pmol/*µ*ℓ |
| TD02 probe | GGGGGGGGG TTT ATA TTT CCTACGA CTTGGGGAGG | 100 pmol/*µ*ℓ |

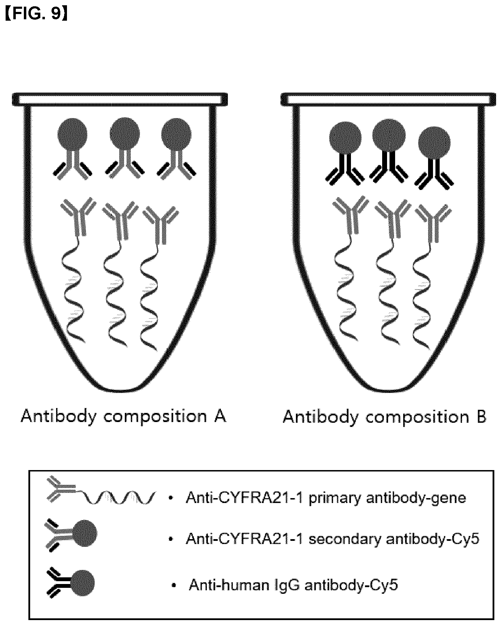

Materials used for immobilizing oligo genes for diagnosing lung cancer and for preparing 9G DNA membrane are listed in Table 6, and the 9G DNA membrane was prepared according to a known method as shown in FIG. 9. (see Examples 2 and 3 of Korean Patent Registration No. 10-0883763)

### <Example 5> Detection of CYFRA21-1 antigen protein and anti-CYFRA21-1 autoantibody-antigen conjugate

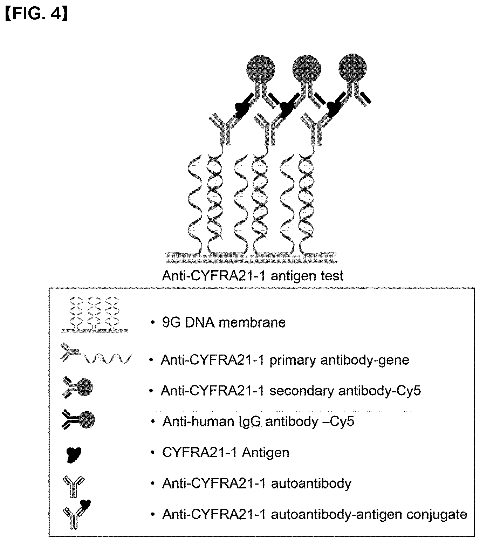

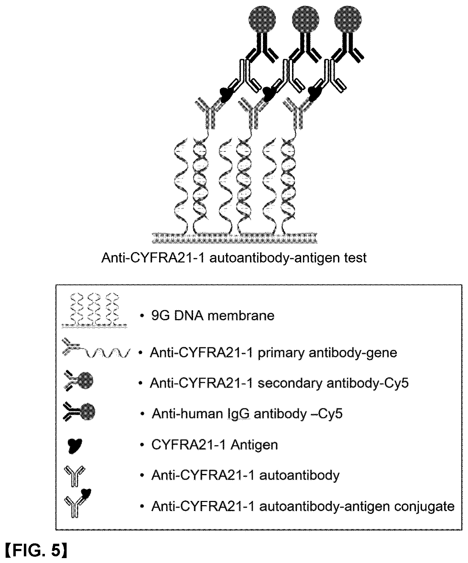

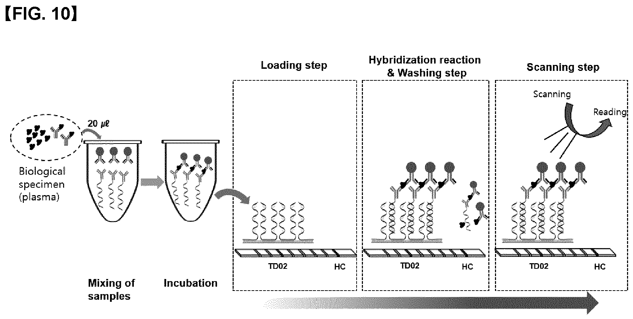

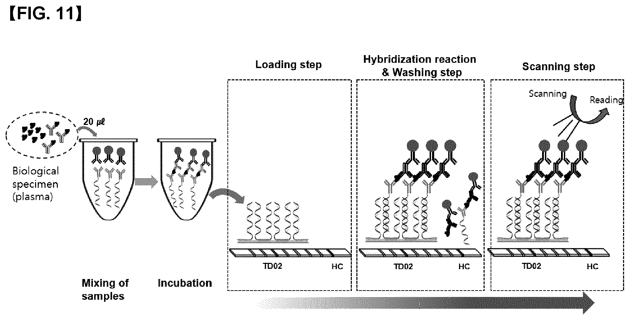

The concept of the method for detecting the CYFRA21-1 antigen protein and the anti-CYFRA21-1 autoantibody-antigen conjugate is schematically illustrated in FIGS. 4 and 5, and the experimental procedure in FIGS. 10 and 11.

### 1) Preparation of blood specimen and material used

As the normal blood and the cancer patient specimen, 200 normal specimens and 50 lung cancer (stages I to IV) specimens were used, and a specimen (IRB#KIRAMS2018-10-006, 2018-06-016) available from the Korea Cancer Center Hospital was used. As a cancer patient specimen, a plasma specimen from a patient clearly diagnosed as lung cancer by biopsy was used. As a normal specimen, a specimen from normal human participated in the health examination was used. The materials used in the experiment are shown in Table 7 below.

**[Table 7]**

| **Material used** | |
|---|---|
| **for detecting CYFRA21-1 antigen** | **for detecting anti-CYFRA21-1 autoantibody-antigen** |
| Blood specimen (20 *µ*ℓ) | Blood specimen (20 *µ*ℓ) |
| Antibody composition A (100 *µ*ℓ) | Antibody composition B (100 *µ*ℓ) |
| R buffer (60 *µ*ℓ) | R buffer (60 *µ*ℓ) |
| W buffer (180 *µ*ℓ) | W buffer (180 *µ*ℓ) |
| 9G DNA membrane for detecting antigen | 9G DNA membrane for detecting autoantibody-antigen |

### 2) Specimen mixing and incubation

### 2-1) For detecting CYFRA21-1 antigen

20 µl of the standard material was added to a 0.2 ml-sized microtube for detecting antigen, and 100 µl of antibody composition A prepared as shown in Table 7 was mixed, and incubated at room temperature for 11 minutes.

### 2-2) For detecting anti-CYFRA21-1 autoantibody-antigen

20 µl of a standard material was added to a 0.2 ml-sized microtube for detecting autoantibody-antigen, and 100 µl of antibody composition B prepared as shown in Table 7 was mixed, and incubated at room temperature for 11 minutes.

### 3) Specimen loading and hybridization reaction

### 3-1) For detecting CYFRA21-1 antigen

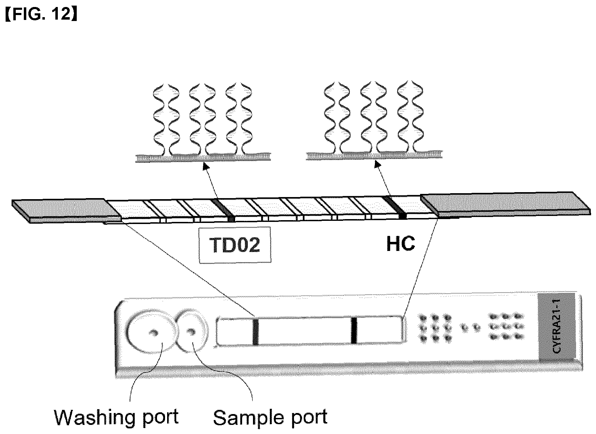

60 µl of R buffer was added to a 0.2 ml-sized microtube for detecting antigen containing 120 µl of the reactant, and then all specimens were taken and 180 µl was loaded on the sample port of the 9G DNA membrane shown in FIG. 12 for antigen-detection, and then subjected to hybridization reaction at room temperature for 11 minutes.

### 3-2) For detecting anti-CYFRA21-1 autoantibody-antigen

60 µl of R buffer was added to a 0.2 ml-sized microtube for detecting autoantibody-antigen containing 120 µl of the reactant, and then all specimens were taken and 180 µl was loaded into a sample port of the 9G DNA membrane shown in FIG. 12 for detecting autoantibody-antigen, and then subjected to a hybridization reaction at room temperature for 11 minutes.

### 4) Washing and scanning

### 4-1) For detecting CYFRA21-1 antigen

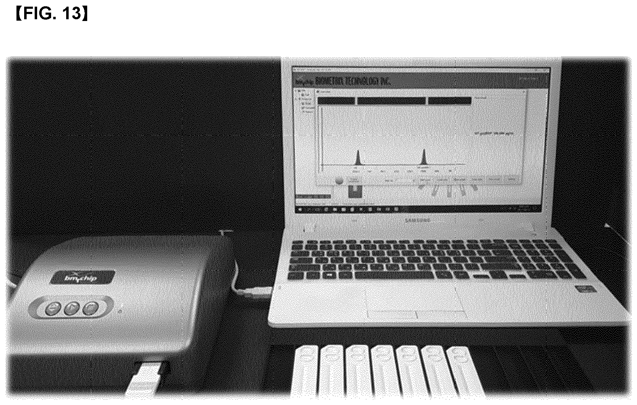

180 µl of W buffer was loaded into a washing port of the 9G DNA membrane for detecting antigen, washed at room temperature for 11 minutes, and read using a 1D scanner as shown in FIG. 13.

### 4-2) For detecting anti-CYFRA21-1 autoantibody-antigen

180 µl of W buffer was loaded into the washing port of the 9G DNA membrane for detecting autoantibody-antigen, and then washed at room temperature for 11 minutes, and then read using a 1D scanner as shown in FIG. 13.

### 5) Analysis of results

### 5.1) Result of fluorescence sensitivity for detecting CYFRA21-1 antigen

Fluorescence sensitivity for detection of CYFRA21-1 antigen in blood specimens from normal human and lung cancer patients was confirmed using the antibody composition A, and is shown in Table 8.

**[Table 8]**

| CYFRA21-1 | Blood specimen from normal human | Blood specimen from lung cancer patient |
|---|---|---|
| A. Fluorescence sensitivity for antigen | 26745 | |

The average value of the fluorescence sensitivity for the CYFRA21-1 antigen was found to be 26745 in blood specimens from normal human, and 19935 in blood specimens from lung cancer patients.

### 5.2) Fluorescence sensitivity for detecting anti-CYFRA21-1 autoantibody-antigen

The fluorescence sensitivity of anti-CYFRA21-1 autoantibody-antigen detection for blood specimens from normal human and lung cancer patients was confirmed using antibody composition B, and is shown in Table 9.

**[Table 9]**

| CYFRA21-1 | Blood specimen from normal human | Blood specimen from lung cancer patients |
|---|---|---|
| B. Fluorescence sensitivity for autoantibody-antigen | 26862 | 47167 |

It was confirmed that the average value of the fluorescence sensitivity for CYFRA21-1 autoantibody-antigen appeared at 26862 in a blood specimen from normal human and at 47167 in a blood specimen from lung cancer patients.

### 5.3) Confirmation of anti-CYFRA21-1 autoantibody-antigen/CYFRA21-1 antigen ratio in blood specimens

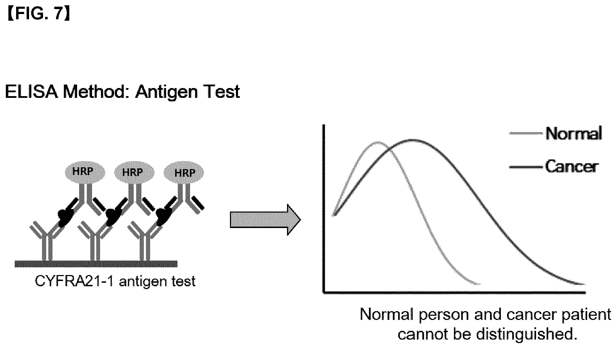

As shown in FIG. 7, the tumor marker CYFRA21-1 antigen test alone cannot differentiate between normal and cancer. Most cancer-related antigens (tumor markers) show a quantitative increase in blood when cancer occurs. The quantitative change in cancer stages 0 to III is very small, and the difference in quantitative change does not appear until the fourth stage is reached. Therefore, it is generally not easy to distinguish between normal and cancer, and in the case of cancer stages 0 to I, it cannot be distinguished even more.

Anti-CYFRA21-1 autoantibody is an autoantibody that reacts specifically when the CYFRA21-1 antigen against lung cancer is present, and in the cancer stages 0 to I, the quantitative increase is 5-10 times higher than the normal.

Therefore, for distinguishing between normal and cancer, it can be determined to be cancer if the ratio of anti-CYFRA21-1 autoantibody to CYFRA21-1 antigen is more than a certain value (cut off) compared to normal.

**[Table 10]**

| CYFRA21-1 | Blood specimen from normal human | Blood specimen from lung cancer patients |
|---|---|---|
| B. Fluorescence sensitivity for autoantibody-antigen | 26862 | 47167 |
| A. Fluorescence sensitivity for antigen | 26745 | 19935 |
| B/A ratio | 1.03 | 2.15 |

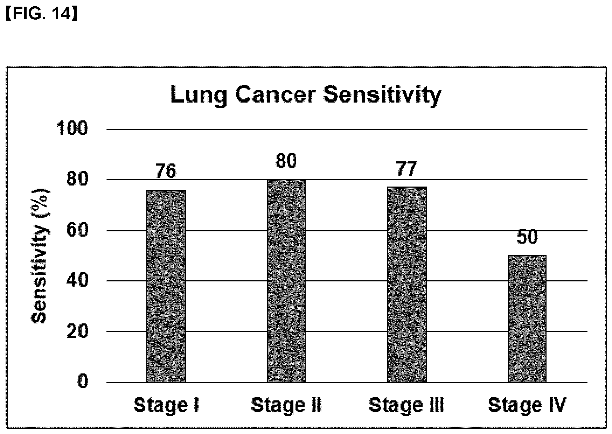

The fluorescence sensitivity results are shown in Table 10, and the analysis results according to the cancer stage are shown in FIG. 14. The ratio (B/A) of anti-CYFRA21-1 autoantibody-antigen conjugate / antigen was found to be 1.03 for normal and 2.15 for lung cancer patients, respectively. Based on the cut off value 1.5 of the B/A ratio, the value of 1.5 or less was determined as normal, and the value of 1.5 or more was determined as lung cancer. A specificity of 91.7% was obtained in 200 normal humans, and sensitivity of stage I 76%, stage II 80%, stage III 77%, and stage IV 50% were obtained in 50 cancer patients.

In cancer stages 0 to I, which is the period when autoantibody rapidly increases, the effect appears to be very high at a sensitivity level of 76%, which proved to be effective in developing a kit for early diagnosis of lung cancer.

A brief description of terms frequently used herein is given below, but these are provided to aid in the understanding of the present disclosure, and should not be used for the purpose of limiting or restricting the scope of the present disclosure.
1. 9G membrane: a glass fiber membrane in which the surface on which oligo genes containing 9 consecutive guanines are immobilized is modified with a calixarene derivative
2. 9G DNA membrane: a glass fiber membrane in which oligo genes containing 9 consecutive guanines are immobilized
3. Antigen (Ag): a material that causes an immune response to generate an antibody, which is generally all kinds of materials that are considered foreign substances in the body.
4. CYFRA21-1 antigen: lung cancer-related tumor marker
5. Anti-CYFRA21-1 primary antibody-gene: antibody-gene conjugate in which a gene is bound to anti-CYFRA21-1 primary antibody
6. Anti-CYFRA21-1 secondary antibody-Cy5: antibody-detection marker conjugate in which Cy5-dye (fluorescent detection marker) is bound to anti-CYFRA21-1 secondary antibody
7. Autoantibody (AAb): It refers to an antibody that reacts specifically with one's biological components, and is also called an autoantibody.
8. Anti-CYFRA-21-1 autoantibody: an antibody that specifically reacts with the CYFRA21-1 antigen, which is a lung cancer-related protein.
9. Anti-CYFRA-21-1 autoantibody-antigen conjugate: a form in which anti-CYFRA-21-1 autoantibody and CYFRA21-1 antigen are bound
10. Anti-Human IgG antibody: an antibody that specifically react with human IgG antibody
11. Anti-human IgG antibody-Cy5: antibody-fluorescent marker conjugate in which Cy5-dye (fluorescent marker) is attached to anti-human IgG antibody

## Claims

1. A immunological composition for diagnosing lung cancer comprising:
an antibody composition A containing an anti-CYFRA21-1 primary antibody-gene and an anti-CYFRA21-1 secondary antibody-detection marker; and
an antibody composition B containing an anti-CYFRA 21-1 primary antibody-gene and an anti-human IgG antibody-detection marker.

2. The immunological composition for diagnosing lung cancer according to claim 1, wherein the detection marker is any one selected from the group consisting of a chromogenic enzyme, a fluorescent material, a fluorescent bead, a radioactive isotope, and a colloid.

3. The immunological composition for diagnosing lung cancer according to claim 2, wherein the detection marker is selected from a fluorescent material.

4. A method for diagnosing lung cancer in a human biological specimen using the immunological composition for diagnosing lung cancer according to claim 1, the method comprising the steps of:
(A) measuring a presence or absence or a concentration of <CYFRA 21-1 antigen> in the biological specimen by using an antibody composition A containing <anti-CYFRA21-1 primary antibody-gene> and <anti-CYFRA21-1 secondary antibody-detection marker>,
(B) measuring a presence or absence or a concentration of <anti-CYFRA 21-1 autoantibody-antigen conjugate> in the biological specimen by using an antibody composition B containing <anti-CYFRA 21-1 primary antibody-gene> and <anti-human IgG antibody-detection marker>; and
(C) determining normal and lung cancer in the biological specimen from the ratio of the concentration obtained in the above (a) and the concentration obtained in the above (b).

5. The method for diagnosing lung cancer according to claim 4, wherein, in the above (a), <anti-CYFRA21-1 primary antibody-gene> and <anti-CYFRA21-1 secondary antibody-detection marker> contained in the antibody composition A are bound to <CYFRA21-1 antigen> in a biological specimen to form <anti-CYFRA21-1 primary antibody-gene>: <CYFRA21-1 antigen>: <anti-CYFRA21-1 secondary antibody-detection marker> complex, thereby measuring the presence or absence or the concentration of <CYFRA21-1 antigen>.

6. The method for diagnosing lung cancer according to claim 4, wherein, in the above (b), <anti-CYFRA21-1 primary antibody-gene> and <anti-human IgG antibody-detection marker> contained in the antibody composition B are bound to <anti-CYFRA21-1 autoantibody-antigen conjugate> in the biological specimen to form <anti-CYFRA21-1 primary antibody-gene>: <anti-CYFRA21-1 autoantibody-antigen>: <anti-human IgG antibody-detection marker> complex, thereby measuring the presence or absence or the concentration of <anti-CYFRA21-1 autoantibody-antigen conjugate>.

7. The method for diagnosing lung cancer according to claim 4, wherein, in the above (c), it determines normal or lung cancer in the biological specimen using the ratio of <anti-CYFRA21-1 primary antibody-gene>: <CYFRA21-1 antigen>: <anti-CYFRA21-1 secondary antibody-detection marker> complex and <anti-CYFRA21-1 primary antibody-gene>: <anti-CYFRA21-1 autoantibody-antigen>: <anti-human IgG antibody-detection marker> complex.

8. The method for diagnosing lung cancer according to claim 4, wherein the biological specimen is any one selected from the group consisting of whole blood, serum, plasma, saliva, urine, sputum, lymph fluid, cerebrospinal fluid, and cell fluid.

9. The method for diagnosing lung cancer according to claim 4, wherein the complexes of antigen-antibody complex formed in the above (a) and (b) are immobilized on a solid substrate.

10. The method for diagnosing lung cancer according to claim 9, wherein the solid substrate includes a 9G membrane or a 9G DNA membrane.

11. The method for diagnosing lung cancer according to claim 4, wherein the antigen specific for the anti-CYFRA21-1 autoantibody is a CYFRA21-1 protein.

12. The method for diagnosing lung cancer according to claim 4, wherein the detection marker is any one selected from the group consisting of a chromogenic enzyme, a fluorescent material, a fluorescent bead, a radioactive isotope, and a colloid.

13. The composition for diagnosing lung cancer according to claim 12, wherein the detection marker is selected from a fluorescent material.

14. A diagnostic kit for lung cancer comprising the immunological composition for diagnosing lung cancer according to claim 1 and a biochip.

15. The diagnostic kit for lung cancer according to claim 14, wherein the biochip comprises a 9G membrane or a 9G DNA membrane.
